# FASCICULE DE BREVET EUROPEEN

(11) **EP 0 691 951 B1**
(45) Date de publication et mention de la délivrance du brevet: **10.06.1998**
(21) Numéro de dépôt: 94911225.4
(22) Date de dépôt: 25.03.1994
(51) Int. Cl.: C07C 269/04, C07C 231/02, C07D 305/14

(54) **PROCEDE DE PREPARATION DE DERIVES DE LA BETA-PHENYLISOSERINE**
VERFAHREN ZUR HERSTELLUNG VON BETA-PHENYLISOSERIN-DERIVATEN
METHOD FOR THE PREPARATION OF BETA-PHENYLISOSERINE DERIVATIVES

(30) Priorité: 29.03.1993 FR 9303575
(43) Date de publication de la demande: 17.01.1996
(73) Titulaire: RHONE-POULENC RORER S.A., 92160 Antony (FR)
(72) Inventeur: DIDIER, Eric, F-75013 Paris (FR); LEON, Patrick, F-69160 Tassin-la-Demi-Lune (FR)
(74) Mandataire: Pilard, Jacques
(86) Numéro de dépôt international: FR9400339
(87) Numéro de publication internationale: WO9422813

(56) Documents cités:
- EP-A- 0 414 610
- TETRAHEDRON LETT. (TELEAY,00404039);90; VOL.31 (44); PP.6429-32 UNIV. PAIS VASCO;FAC. QUIM.; SAN SEBASTIAN; 20080; SPAIN (ES) Palomo C et al 'Highly stereoselective synthesis of.alpha.-hydroxy.beta.-amino acids through.beta.-lactams: application to the synthesis of the taxol and bestatin side chains and related systems'

## Description

La présente invention concerne un procédé de préparation de dérivés de la β-phénylisosérine de formule générale: 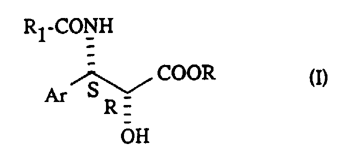 dans laquelle :
Ar représente un radical aryle et R représente un atome d'hydrogène ou un radical alcoyle éventuellement substitué par un radical phényle et R₁ représente un radical phényle éventuellement substitué par un ou plusieurs atomes ou radicaux, identiques ou différents, choisis parmi les atomes d'halogène et les radicaux alcoyles, hydroxy, alcoxy, alcanoyle, alcanoyloxy, nitro, amino, alcoylamino, dialcoylamino, carbamoyle ou trifluorométhyle, les radicaux alcoyles et les portions alcoyles des autres radicaux contenant 1 à 4 atomes de carbone, ou bien R₁ représente un radical R₂-O- dans lequel R₂ représente :
- un radical alcoyle droit ou ramifié contenant 1 à 8 atomes de carbone, alcényle contenant 3 à 6 atomes de carbone, cycloalcoyle contenant 3 à 6 atomes de carbone, cycloalcényle contenant 4 à 6 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 4 à 6 atomes de carbone, alcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons et éventuellement substitué par un ou plusieurs radicaux alcoyles contenant 1 à 4 atomes de carbone.

De préférence, Ar représente un radical phényle ou *α-* ou β-naphtyle éventuellement substitué par un ou plusieurs atomes ou radicaux choisis parmi les atomes d'halogène (fluor, chlore, brome, iode) et les radicaux alcoyles, aryles, aralcoyles, alcoxy, alcoylthio, aryloxy, arylthio, hydroxy, hydroxyalcoyle, mercapto, formyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone et que les radicaux aryles sont des radicaux phényles ou *α-* ou β-naphtyles.

Plus particulièrement, Ar représente un radical phényle éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

Plus particulièrement encore, Ar représente un radical phényle éventuellement substitué par un atome de chlore ou de fluor ou par un radical alcoyle (méthyle), alcoxy (méthoxy), dialcoylamino (diméthylamino), ou acylamino (acétylamino).

Il est connu selon le brevet EP-A-414 610 un procédé de préparation énantiosélectif de dérivés de formule générale (I) qui consiste à traiter un composé de formule: 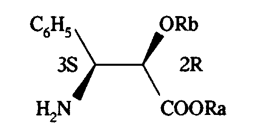 dans laquelle Rₐ représente un atome d'hydrogène ou un groupe alkyle et R_{b} un atome d'hydrogène ou un groupe protecteur de la fonction hydroxy, par un agent acylant. Le produit présentant la bonne structure énantiomère de départ étant obtenu par l'intermédiaire d'un hydroxy azoture.

Il est aussi connu d'après l'article de Tetrahedron Letters, vol. 31, N° 44, page 6429 d'acyler un dérivé consistant en un mélange des 2 énantiomères de la forme cis du dérivé de formule ci-dessus pour lequel R_{b} représente l'hydrogène. La phénylisosérine de bonne configuration (3S,2R) n'est jamais obtenue.

Selon la présente invention, les produits de formule générale (I) sont obtenus par action d'un anhydride de formule générale :

(R₁-CO)₂O (II)

dans laquelle R₁ est défini comme précédemment, en présence d'hydrogène sur un produit de formule générale : 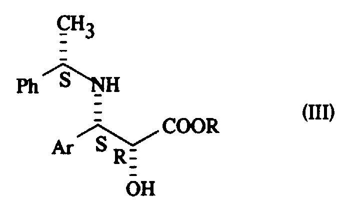 dans laquelle Ar et R sont définis comme précédemment et Ph représente un radical phényle ou *α-* ou β-naphtyle éventuellement substitué par un ou plusieurs atomes ou radicaux choisis parmi les atomes d'halogène (fluor, chlore, brome, iode) et les radicaux alcoxy contenant 1 à 4 atomes de carbone, alcoylthio contenant 1 à 4 atomes de carbone, dialcoylamino dont chaque partie alcoyle contient 1 à 4 atomes de carbone ou nitro. Plus particulièrement, Ph représente un radical phényle éventuellement substitué par un ou plusieurs radicaux, identiques ou différents, choisis parmi les radicaux méthoxy, méthylthio, méthylamino, diméthylamino ou nitro.

Généralement, le procédé est mis en oeuvre en faisant réagir l'anhydride de formule générale (II) en présence d'hydrogène, éventuellement sous pression, et d'un catalyseur d'hydrogénolyse sur le produit de formule générale (III) dans un solvant organique à une température comprise entre 0°C et la température de reflux du mélange réactionnel.

L'hydrogène nécessaire à la mise en oeuvre du procédé peut être éventuellement fourni par un composé qui libère de l'hydrogène par réaction chimique ou par décomposition thermique tel que le formiate d'ammonium.

Selon un mode préféré de mise en oeuvre du procédé, on opère sous une pression d'hydrogène qui peut être comprise entre 1 et 50 bars.

Le catalyseur est de préférence constitué par du palladium sur charbon contenant 1 à 10 % en poids de palladium ou le dihydroxyde de palladium sur charbon contenant jusqu'à 10 % en poids de palladium.

Le procédé est mis en oeuvre dans un solvant organique ou dans un mélange de solvants organiques.

Comme solvants organiques qui conviennent particulièrement bien peuvent être cités les alcools aliphatiques contenant 1 à 4 atomes de carbone tels que le méthanol, l'éthanol ou l'isopropanol, les acides aliphatiques tels que l'acide acétique et les hydrocarbures aromatiques tels que le benzène, le toluène ou les xylènes.

De préférence, le procédé est mis en oeuvre dans un alcool aliphatique contenant 1 à 4 atomes de carbone et, plus particulièrement dans le méthanol ou dans un hydrocarbure aromatique et plus particulièrement dans le toluène.

Le procédé selon l'invention peut aussi être mise en oeuvre en passant intermédiairement par un produit de formule générale : 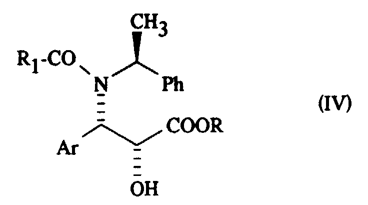 dans laquelle Ar, R, R₁ et Ph sont définis comme précédemment qui est hydrogénolysé en produit de formule générale (I).

Généralement, le produit de formule générale (IV) est obtenu par action de l'anhydride de formule générale (II) sur le produit de formule générale (III) en absence d'hydrogène et de catalyseur d'hydrogénation en opérant de préférence dans un hydrocarbure aromatique.

Généralement l'hydrogénolyse du produit de formule générale (IV) est effectuée au moyen d'hydrogène en présence d'un catalyseur tel que défini comme précédemment en opérant de préférence dans un alcool aliphatique contenant 1 à 4 atomes de carbone tel que le méthanol.

Il n'est pas nécessaire d'isoler préalablement le produit de formule générale (IV) avant de procéder à l'hydrogénolyse.

Le produit de formule générale (III) peut être obtenu par hydrolyse ou alcoolyse d'un produit de formule générale : 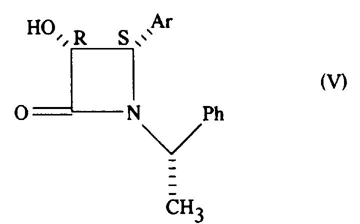 dans laquelle Ar et Ph sont définis comme précédemment.

Il est particulièrement avantageux d'effectuer une alcoolyse au moyen d'un alcool de formule générale R-OH dans laquelle R est défini comme précédemment en opérant en milieu acide.

De préférence, on effectue l'alcoolyse au moyen de méthanol en présence d'un acide minéral fort tel que l'acide chlorhydrique.

Il est avantageux d'effectuer l'alcoolyse à une température voisine de la température de reflux du mélange réactionnel.

Le produit de formule générale (V) peut être obtenu par saponification ou hydrogénolyse d'un produit de formule générale : 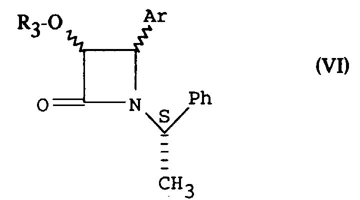 dans laquelle Ar et Ph sont définis comme précédemment et R₃ représente un groupement protégeant la fonction alcool sous forme d'ester ou d'ether, suivie de la séparation du diastéréoisomère 3R,4S de formule générale (IV) des autres diastéréoisomères.

Plus particulièrement R₃ représente un radical alcoyle, phénylalcoyle ou phényle ou un radical R'₃-CO dans lequel R'₃ représente un radical alcoyle, phénylalcoyle ou phényle.

Généralement, lorsque la fonction alcool est protégée sous forme d'ester, on effectue une saponification au moyen d'une base minérale ou organique telle que l'ammoniaque, la lithine, la soude ou la potasse dans un solvant convenable.

Comme solvant, on utilise de préférence un milieu hydroorganique tel qu'un mélange méthanol-eau ou tétrahydrofuranne-eau. La réaction est mise en oeuvre à une température comprise entre -10 et +20°C.

Généralement, lorsque la fonction alcool est protégée sous forme d'éther, on effectue une hydrogénolyse au moyen d'hydrogène, éventuellement généré in situ, par exemple, par décomposition du formiate d'ammonium, en présence d'un catalyseur tel que le palladium sur noir contenant de 1 à 10 % de palladium (p/p).

La séparation du diastéréoisomère (3R,4S) peut être effectuée par cristallisation sélective dans un solvant organique convenable tel que l'acétate d'éthyle éventuellement en présence d'un hydrocarbure aliphatique tel que l'hexane ou par chromatographie sur silice.

Le produit de formule générale (VI) peut être obtenu par cycloaddition d'une imine de formule générale : 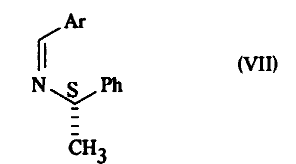 dans laquelle Ar et Ph sont définis comme précédemment, sur un halogénure d'acide de formule générale: 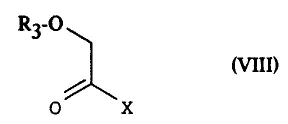 dans laquelle R₃ est défini comme précédemment et X représente un atome d'halogène tel qu'un atome de brome ou de chlore.

Généralement la réaction est effectuée à une température comprise entre -20 et 50°C, de préférence au voisinage de 0°C en présence d'une base choisie parmi les amines tertiaires (triéthylamine, N-méthyl-morpholine) ou la pyridine dans un solvant organique choisi parmi les hydrocarbures aliphatiques éventuellement halogénés tels que le chlorure de méthylène ou le chloroforme et les hydrocarbures aromatiques tels que le benzène, le toluène ou les xylènes.

Le produit de formule générale (V) peut être obtenu dans les conditions décrites par M. Furukawa et coll., Chem. Pharm. Bull., 25(1), 181-184 (1977).

Le produit de formule générale (I) dans laquelle R représente un atome d'hydrogène peut aussi être obtenu par saponification d'un produit de formule générale (I) dans laquelle R représente un radical alcoyle contenant 1 à 4 atomes de carbone, éventuellement substitué par un radical phényle ou un radical phényle.

Généralement la saponification est réalisée au moyen d'une base minérale telle qu'un hydroxyde de métal alcalin (lithine, soude), un carbonate ou un bicarbonate alcalin (bicarbonate de sodium, carbonate de potassium) en milieu hydroalcoolique tel qu'un mélange méthanol-eau en opérant à une température comprise entre 10 et 40°C, de préférence voisine de 25°C.

Les produits de formule générale (I) sont particulièrement utiles pour préparer les dérivés du taxane de formule générale : 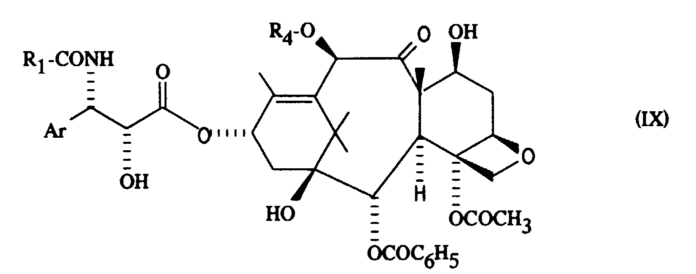 dans laquelle Ar et R₁ sont définis comme précédemment, et R₄ représente un atome d'hydrogène ou un radical acétyle qui présentent des propriétés antitumorales et antileucémiques remarquables.

Le produit de formule générale (IX) dans laquelle Ar représente un radical phényle, R₁ représente un radical phényle et R₄ représente un radical acétyle est connu sous le nom de taxol et celui pour lequel Ar représente un radical phényle, R₁ représente un radical tert.butoxy et R₄ représente un atome d'hydrogène est connu sous le nom de Taxotère.

Les dérivés du taxane de formule générale (IX) peuvent être obtenus par action d'un acide de formule générale : 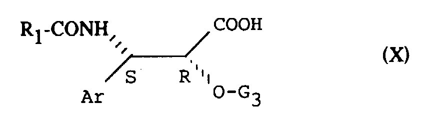 dans laquelle Ar et R₃ sont définis comme précédemment et G₃ représente un groupement protecteur de la fonction hydroxy tel qu'un radical méthoxyméthyle, (éthoxy-1) éthyle, benzyloxyméthyle, (β-triméthylsilyléthoxy) méthyle, tétrahydropyrannyle, trichloro-2,2,2 éthoxyméthyle ou trichloro-2,2,2 éthoxycarbonyle, éventuellement sous forme d'halogénure, d'anhydride ou d'anhydride mixte, sur un dérivé du taxane de formule générale : 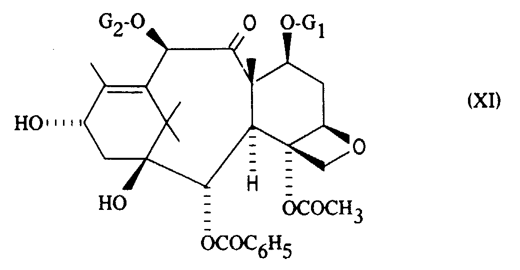 dans laquelle G₁ représente un groupement protecteur de la fonction hydroxy tel qu'un radical trichloro-2,2,2 éthoxycarbonyle ou trialcoylsilyle, dialcoylarylsilyle, alcoyldiarylsilyle ou triarylsilyle dans lequel chaque partie alcoyle contient 1 à 4 atomes de carbone et chaque partie aryle représente de préférence un radical phényle et G₂ représente un radical acétyle ou un groupement protecteur de la fonction hydroxy tel qu'un radical trichloro-2,2,2 éthoxycarbonyle pour donner un produit de formule générale : 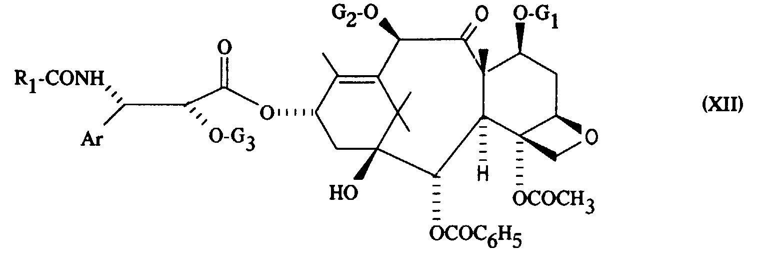 dans laquelle Ar, R₁, G₁, G₂ et G₃ sont définis comme précédemment, suivie du remplacement des groupements G₁, G₂ et G₃ par des atomes d'hydrogène.

Généralement l'estérification est effectuée en présence d'un agent de condensation tel qu'un carbodiimide comme le dicyclohexylcarbodiimide ou un carbonate réactif comme le pyridyl-2 carbonate et d'un agent d'activation tel qu'une aminopyridine comme la diméthylamino-4 pyridine ou la pyrrolidino-4 pyridine en opérant dans un solvant organique tel qu'un hydrocarbure aromatique (benzène, toluène, xylène, éthylbenzène, isopropylbenzène, chlorobenzène), un éther (tétrahydrofuranne), un nitrile (acétonitrile) ou un ester (acétate d'éthyle) à une température comprise entre 0 et 90°C.

Le remplacement des groupements protecteurs G₁, G₂ et G₃ par des atomes d'hydrogène est effectué généralement par traitement par le zinc en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone en présence de zinc lorsque l'un des groupements protecteurs représente un radical trichloro-2,2,2 éthoxycarbonyle ou par traitement en milieu acide lorsque l'un des groupements protecteurs représente un radical silylé.

L'acide de formule générale (X) peut être obtenu par saponification d'un ester de formule générale : 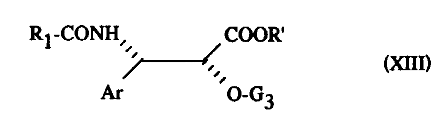 dans laquelle Ar, R₁ et G₃ sont définis comme précédemment et R' représente un radical alcoyle contenant 1 à 4 atomes de carbone, éventuellement substitué par un radical phényle, ou un radical phényle, au moyen d'une base minérale telle qu'un hydroxyde de métal alcalin (lithine, soude), un carbonate ou un bicarbonate de métal alcalin (bicarbonate de sodium, carbonate de potassium) en milieu hydroalcoolique tel qu'un mélange méthanol-eau en opérant à une température comprise entre 10 et 40°C, de préférence voisine de 25°C.

Le produit de formule générale (XIII) peut être obtenu dans les conditions habituelles de préparation des éthers, et plus particulièrement selon les procédés décrits par J-N. Denis et coll., J. Org. Chem., 51, 46-50 (1986) à partir d'un produit de formule générale (I) dans laquelle R représente un radical alcoyle contenant 1 à 4 atomes de carbone éventuellement substitué par un radical phényle.

Les dérivés du taxane de formule générale (IX) peuvent aussi être obtenus en transformant d'abord un produit de formule générale (I) dans laquelle R représente un atome d'hydrogène en dérivé de l'oxazolidine de formule générale : 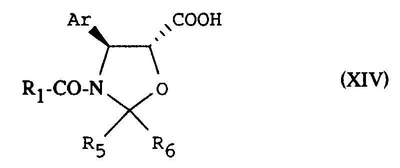 dans laquelle Ar et R₁ sont définis comme précédemment et R₅ et R₆, identiques ou différents, représentent un atome d'hydrogène ou un radical alcoyle contenant 1 à 4 atomes de carbone ou un radical aryle de préférence un radical phényle éventuellement substitué par un ou plusieurs radicaux alcoxy contenant 1 à 4 atomes de carbone, ou bien R₅ représente un radical alcoxy contenant 1 à 4 atomes de carbone ou un radical trihalométhyle tel que trichlorométhyle et R₆ représente un atome d'hydrogène, ou bien R₅ et R₆ forment ensemble avec l'atome de carbone auquel ils sont liés un cycle ayant 4 à 7 chaînons, puis en estérifiant le dérivé du taxane de formule générale (XI) au moyen de l'acide de formule générale (XIV) pour obtenir un produit de formule générale : 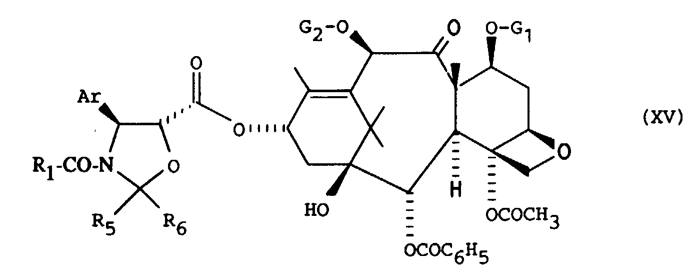 dans laquelle Ar, G₁, G₂, R₁, R₅ et R₆ sont définis comme précédemment, qui est transformé en dérivé du taxane de formule générale (IX) en passant intermédiairement, lorsque R₅ et R₆, identiques ou différents, représentent un radical alcoyle contenant là 4 atomes de carbone, ou un radical aryle de préférence un radical phényle éventuellement substitué, ou bien R₅ représente un radical trihalométhyle ou un radical phényle substitué par un radical trihalométhyle et R₆ représente un atome d'hydrogène, ou bien R₅ et R₆ forment ensemble avec l'atome de carbone auquel ils sont liés un cycle ayant 4 à 7 chaînons, par un dérivé du taxane de formule générale : 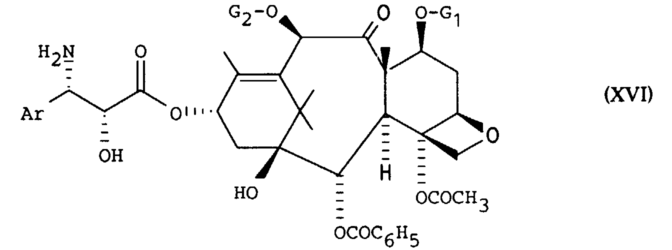 qui est acylé au moyen de chlorure de benzoyle ou d'un produit de formule générale :

R₁-O-CO-X (XVII)

dans laquelle R₁ est défini comme précédemment et X représente un atome d'halogène (fluor, chlore) en opérant par exemple dans les conditions décrites dans la demande PCT WO 9209589, avant d'obtenir un produit de formule générale : 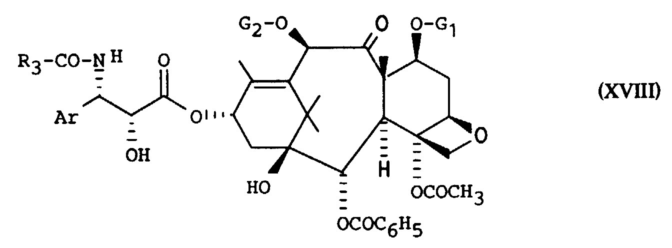 dont les groupements protecteurs G₁ et G₂ sont remplacés par des atomes d'hydrogène dans les conditions décrites précédemment.

Les exemples suivants illustrent l'invention.

### EXEMPLE 1

A une solution de 250 g d'hydroxy-2 [phényl-1-(S)]éthylamino-3 phényl-3 propionate-(2R,3S) de méthyle dans 1000 cm3 de méthanol, on ajoute, à, 25°C, une solution de 198 g de diterbutyldicarbonate dans 250 cm3 de méthanol et 50 g de charbon palladié à 10 % (50 % d'humidité). La solution est alors hydrogénée sous pression atmosphérique d'hydrogène à 25°C pendant 10 heures. Le catalyseur est filtré sur un verre fritté recouvert de Clarcel et le catalyseur est lavé deux fois avec 110 cm3 de méthanol. Le filtrat et les eaux de lavage sont rassemblés et refroidis à 0°C. On ajoute alors en une heure et quarante minutes 510 cm3 d'eau déminéralisée. La solution est filtrée sur verre fritté et le produit lavé deux fois avec 200 cm3 d'un mélange méthanol-eau (70-30 en volumes). Après séchage, on obtient 182,5 g de tert-butoxycarbonylamino-3 hydroxy-2 phényl-3 propionate-(2R,3S) de méthyle fondant à 135°C (après recristallisation dans l'oxyde de diisopropyle) et dont les caractéristiques sont les suivantes :
- pouvoir rotatoire : [α]²⁰_{D} = 2,6° (c = 1; méthanol) [α]²⁰_{D} = -7,4° (c = 1,03 ; chloroforme)
- spectre de RMN (200 MHz ; CDCl₃ ; déplacements chimiques δ en ppm ; constantes de couplage J en Hz) : 1,42 (s, 3H : -NH-COOC(CH₃)₃ ; 3,16 (d, 1H, J = 5 : -OH) ; 3,87 (s, 3H : COOCH₃) ; 4,48 (m, 1H: -CHOH); 5,22 (d large, 1H, J = 10,5 : -CHNHCOOC(CH₃)₃) ; 5,39 (d, 1H, J = 10,5 : -NHCOOC(CH₃)₃) ; 7,20 à 7,45 (m, 5H : -C₆H₅).

L'hydroxy-2 [phényl-1-(S)] éthylamino-3 phényl-3 propionate-(2R,3S) de méthyle peut être préparé de la manière suivante :

Une solution de 0,8 g d'hydroxy-3 phényl-4 [phényl-1-(S)] éthyl-1 azétidinone-2-(3R,4S) dans un mélange de 30 cm3 de méthanol et de 6 cm3 d'une solution aqueuse 6N d'acide chlorhydrique est chauffée au reflux (65°C) pendant 20 heures, puis refroidie à une température voisine de 20°C et concentrée à sec sous pression réduite (2,7 kPa) à une température voisine de 40°C. Le résidu est additionné de 20 cm3 d'eau distillée et alcalinisé jusqu'à un pH voisin de 7 par addition d'une solution aqueuse 7,5N d'hydroxyde de sodium puis extrait par 3 fois 25 cm3 de dichlorométhane. Les phases organiques sont réunies, séchées sur sulfate de magnésium, filtrées puis concentrées à sec sous pression réduite (2,7 kPa) à 40°C. On obtient ainsi 0,74 g d'hydroxy-2 [(phényl-1(S)] éthylamino-3 phényl-3 propionate-(2R,3S) de méthyle sous forme d'une huile jaune pâle dont les caractéristiques sont les suivantes :
- pouvoir rotatoire : [α]²⁰_{D} = -22,7° (c = 1,00 ; méthanol)
- spectre de RMN (200 MHz ; CDCl₃)
   δ (ppm) : 1,34 (d, 3H, J = 7Hz : -CCH₃) ; 2,7 (m, 2H : -CNHC- et -OH) ; 3,71 (q,) 1H, J = 7 Hz : -CHNH-) ; 3,84 (s, 3H : -COOCH₃) ; 4,2 (d, 1H, J = 4 Hz : -CHOH-); 4,35 (d, 1H, J = 4 Hz : -CHNH-); 7,20 à 7,45 (m, 5H : -C₆H₅).

L'hydroxy-3 phényl-4 [phényl-1-(S)] éthyl-1 azétidone-2(3R,4S) peut être préparée selon l'une des méthodes suivantes :
1) A un mélange de 120 cm3 d'une solution aqueuse 1N d'hydroxyde de potassium et de 90 cm3 de tétrahydrofuranne on ajoute en 35 minutes, sous agitation et à une température voisine de 0°C, une solution de 3,3 g d'un mélange en proportion molaire 75/25 des deux diastéréoisomères d'acétoxy-3 phényl-4 [phényl-1-(S)] éthyl-1 azétidinone-2 forme A et forme B dans 120 cm3 de tétrahydrofuranne. L'addition terminée le milieu réactionnel est agité à une température voisine de 0°C pendant 1 heure puis additionné de 120 cm3 d'une solution aqueuse saturée d'hydrogénocarbonate de sodium et de 100 cm3 d'eau distillée. La phase aqueuse est séparée par décantation et réextraite par 3 fois 100 cm3 d'acétate d'éthyle. Les phases organiques sont réunies, séchées sur sulfate de magnésium, filtrées puis concentrées à sec sous pression réduite (2,7 kPa) à 40°C. On obtient ainsi 2,8 g de cristaux blancs que l'on recristallise dans 35 cm3 d'un mélange d'acétate d'éthyle et d'hexane (80-20 en volumes) pour donner 1,92 g d'hydroxy-3 phényl-4 [phényl-1-(S)] éthyl-1 azétidinone-2-(3R,4S) sous forme de cristaux blancs fondant à 162°C et dont les caractéristiques sont les suivantes :
- pouvoir rotatoire : [α]²⁰_{D} = +132° (c = 1,08 ; méthanol)
- de RMN (200 MHz ; CDCl₃)
   δ (ppm): 1,41 (d, 3H, J = 7 Hz : -CHCH₃) ; 2,36 (d, 1H, J = 8,5 Hz : -OH); 4,58 (d, 1H, J = 4,5 Hz : -CHC₆H₅) ; 4,90 (dd, 1H, J = 8,5 Hz et 4,5 Hz : -CHOH-); 5,06 (q, 1H, J = 7 Hz : -CHCH₃) ; 7,20 à 7,50 (m, 5H : -C₆H₅).

Le mélange de la forme A et de la forme B de l'acétoxy-3 phényl-4 [phényl-1-(S)] éthyl-1 azétidone-2 peut être préparé de la manière suivante :

A une solution de 14,63 g de N-benzylidène-(phényl-1 éthylamine)-(S) dans 180 cm3 de chloroforme on ajoute, sous agitation et à une température voisine de 20°C, 19,6 cm3 de triéthylamine puis on refroidit le mélange réactionnel jusqu'à une température voisine de -20°C et additionne goutte à goutte, en 75 minutes et en se maintenant à cette température, 5,17 cm3 de chlorure d'acétoxy-2 acétyle dans 90 cm3 de chloroforme. La solution obtenue est agitée pendant 16 heures à une température voisine de 20°C puis additionnée de 300 cm3 d'une solution aqueuse 2,7N d'acide chlorhydrique. La phase organique est séparée par décantation, lavée par 2 fois 300 cm3 d'eau distillée puis par 300 cm3 d'une solution aqueuse saturée d'hydrogénocarbonate de sodium, séchée sur sulfate de magnésium, filtrée puis concentrée à sec sous pression réduite (2,7 kPa) à 40°C. On obtient ainsi 16,5 g d'une huile brune que l'on purifie par chromatographie sur 800 g de silice (0,04-0,063 mm) contenus dans une colonne de 6,8 cm de diamètre [éluant : cyclohexane-acétate d'éthyle (70-30 en volumes)] en recueillant des fractions de 22 cm3. Les fractions 100 à 153 sont réunies et concentrées à sec sous pression réduite (0,27 kPa) à 40°C. On obtient ainsi 10,65 g d'un mélange en proportion molaire 75/25 de deux diastéréoisomères d'acétoxy-3 phényl-4 [phényl-1-(S)] éthyl-1 azétidinone-2 sous forme d'une huile jaune.

Le N-benzylidène-(phényl-1 éthylamine)-(S) peut être préparé selon la méthode décrite par M. Furukawa et coll., Chem. Pharm. Bull., 1977, 25(1), 181-184.

2) En opérant comme précédemment, mais à partir de 100 mg d'un mélange en proportion molaire 70/30 des deux diastéréoisomères d'isobutyryloxy-3 phényl-4 [phényl-1-(S)]éthyl-1 azétidinone-2 forme A et forme B, on obtient 82 mg d'hydroxy-3 phényl-4 [phényl-1-(S)]éthyl-1 azétidinone-2-(3R,4S) sous forme de cristaux blancs fondants à 162°C dont les caractéristiques physiques sont identiques à celles du produit obtenu précédemment.
- Le mélange des formes A et B de l'isobutyryloxy-3 phényl-4 [phényl-1-(S)]éthyl-1 azétidinone-2 peut être préparé en opérant comme précé--demment mais à partir de 1,91 g de N-benzylidène-(phényl-1 éthylamine)-(S) et de 1 g de chlorure d'isobutyryloxy-2 acétyle. On obtient ainsi 1,27 g d'un mélange en proportion molaire : 70/30 de deux diastéréoisomères d'isobutyryloxy-3 phényl-4 [phényl-1-(S)]éthyl-1 azétidinone-2 sous forme d'une huile jaune.
- Le chlorure d'isobutyryloxy-2 acétyle peut être préparé de la manière suivante :
- A une solution de 5 g d'acide glycolique dans 100 cm3 de dichlorométhane, maintenue sous atmosphère d'argon, on ajoute sous agitation et à une température voisine de 20°C 18,3 cm3 de triéthylamine puis on refroidit le mélange réactionnel jusqu'à une température voisine de 5°C et additionne goutte à goutte, en 30 minutes et en se maintenant à cette température, 13,8 cm3 de chlorure d'isobutyryle. La solution obtenue est agitée pendant 3 heures à une température voisine de 20°C. Le précipité apparu est séparé par filtration et lavé par 2 fois 10 cm3 de dichlorométhane. Les filtrats réunis sont lavés par 60 cm3 d'une solution aqueuse saturée de chlorure d'ammonium puis par 30 cm3 d'une solution aqueuse saturée de chlorure de sodium, séchés sur sulfate de magnésium, filtrés puis concentrés à sec sous pression réduite (2,7 kPa) à 40°C. On obtient ainsi 13 g d'une huile jaune à laquelle on ajoute 24 cm3 de chlorure de sulfinyle. La solution obtenue est chauffée au reflux pendant 2,5 heures puis distillée sous pression réduite (0,07 kPa ; 0,5 mmHg). On obtient ainsi 3,4 g de chlorure d'isobutyryloxy-2 acétyle sous forme d'un liquide incolore distillant à 45-50°C sous une pression de 0,07 kPa.

3) A 43 mg d'une dispersion à 10 % de palladium dans du charbon en poudre, on ajoute une solution de 91 mg d'un mélange en proportion molaire 60/40 des deux diastéréoisomères de benzyloxy-3 phényl-4 [phényl-1-(S)]éthyl-1 azétidinone-2 forme A et forme B dans 6 cm3 de méthanol puis 32 mg de formiate d'ammonium. Le mélange réactionnel est maintenu sous agitation et sous atmosphère d'argon pendant 72 heures à une température voisine de 20°C puis on ajoute 56 mg de la dispersion de palladium à 10 % et 128 mg de formiate d'ammonium. Le mélange réactionnel est maintenu sous agitation à la même température pendant 26 heures. Le mélange réactionnel est ensuite filtré sur verre fritté garni de célite. Le verre fritté est lavé par 3 fois 5 cm3 de dichlorométhane puis les filtrats réunis sont concentrés sous pression réduite (2,7 kPa) à une température voisine de 40°C. On obtient ainsi 70 mg de cristaux blancs que l'on purifie par chromatographie sur gel de silice déposé sur plaque (gel de 1 mm d'épaisseur ; plaque de 20 X 20 cm) par fractions de 10 mg. Après localistion aux rayons U.V. de la zone correspondant au produit cherché, cette zone est grattée et la silice est recueillie puis lavée sur verre fritté par 10 fois 5 cm3 de dichlorométhane et par 5 fois 2 cm3 de méthanol. les filtrats sont réunis et concentrés à sec sous pression réduite (0,27 kPa) à 40°C. On obtient ainsi 28 mg d'hydroxy-3 phényl-4 [phényl-1-(S)]éthyl-1 azétidinone-2-(3R,4S) sous forme de cristaux blancs fondants à 162°C dont les caractéristiques physiques sont identiques à celles du produit obtenu précédemment.

Le mélange des formes A et B du benzyloxy-3 phényl-4 [phényl-1-(S)]éthyl-1 azétidinone-2 peut être préparé en opérant comme précédemment mais à partir de 2,0 g de N-benzylidène-(phényl-1 éthylamine)-(S) et de 1,38 g de chlorure de benzyloxy-2 acétyle. On obtient ainsi 1,25 g d'un mélange en proportion molaire : 60/40 de deux diastéréoisomères de benzyloxy-3 phényl-4 [phényl-1-(S)]éthyl-1 azétidinone-2 sous forme d'une huile jaune.

### EXEMPLE 2

Dans un ballon de 25 cm3, on introduit 1 g d'hydroxy-2 [phényl-1-(S)]éthyl amino-3 phényl-3 propionate-(2R,3S) de méthyle, 0,8 g d'anhydride benzoïque, 14 cm3 de toluène et 0,2 g de palladium sur charbon à 10 % (p/p). On purge à l'argon puis on place sous atmosphère d'hydrogène. Le mélange réactionnel est agité pendant 23 heures à 20°C puis pendant 3 heures à 87°C. Après refroidissement et séparation du catalyseur par filtration sur verre fritté, la solution toluénique est neutralisée au moyen d'une solution saturée de bicarbonate de sodium.

La couche organique est lavée par 10 cm3 d'eau. Après séchage et concentration à sec sous pression réduite le résidu est repris par 10 cm3 d'acétone. Après filtration sur filtre Whatman et lavage du filtre par 10 cm3 d'acétone et 10 cm3 de dichlorométhane, les phases organiques sont réunies puis concentrées à sec sous pression réduite. On obtient ainsi 0,8 g d'un produit dont l'analyse par résonance magnétique nucléaire du proton montre qu'il contient 10 % de benzoylamino-3 hydroxy-2 phényl-3 propionate-(2R,3S) de méthyle et 86 % de (N-benzoyl N-phényl-1-(S)éthyl) amino-3 hydroxy-2 phényl-3 propionate-(2R,3S) de méthyle.

### EXEMPLE 3

Dans un ballon de 25 cm3, on introduit 0,51 g d'hydroxy-2 [phényl-1-(S)] éthyl-amino-3 phényl-3 propionate-(2R,3S) de méthyle, 0,39 g d'anhydride benzoïque et 7 cm3 de toluène. On chauffe pendant 3 heures à 80°C. Le toluène est éliminé par distillation sous pression réduite. On ajoute alors 7 cm3 de méthanol et 0,128 g de palladium sur charbon à 10 % contenant 50 % d'eau. On chauffe pendant 3 heures 15 minutes au reflux. Après refroidissement, le catalyseur est séparé par filtration sur verre fritté. Le filtre est rincé par de l'acétone. Les phases organiques sont filtrées sur filtre Whatman puis concentrées à sec sous pression réduite. Le résidu est repris par 15 cm3 d'un mélange acétate d'éthyle-toluène. La solution est lavée par une solution saturée de bicarbonate de sodium puis par 2 fois 10 cm3 d'eau. Après séchage, filtration et concentration à sec sous pression réduite, on obtient 0,4 g d'un produit dont l'analyse par résonance magnétique nucléaire du proton montre qu'il contient 56 % de benzoylamino-3 hydroxy-2 phényl-3 propionate-(2R,3S) de méthyle et 33 % de [N-benzoyl N-phényl-1-(S)éthyl] amino-3 hydroxy-2 phényl-3 propionate-(2R,3S) de méthyle.

## Revendications

1. Procédé de préparation de dérivés de la (β-phénylisosérine de formule générale : 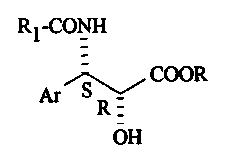 dans laquelle Ar représente un radical aryle, R représente un atome d'hydrogène ou un radical alcoyle éventuellement substitué par un radical phényle et R₁ représente un radical phényle éventuellement substitué ou un radical R₂-O- dans lequel R₂ représente
- un radical alcoyle droit ou ramifié contenant 1 à 8 atomes de carbone, alcényle contenant 3 à 6 atomes de carbone, cycloalcoyle contenant 3 à 6 atomes de carbone, cycloalcényle contenant 4 à 6 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en 4 par un radical alcoyle contenant 1 à 4 atomes de carbone ou par un radical phénylalcoyle dont la partie alcoyle contient 1 à 4 atomes de carbone), cycloalcoyle contenant 4 à 6 atomes de carbone, alcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons et éventuellement substitué par un ou plusieurs radicaux alcoyles contenant 1 à 4 atomes de carbone,
caractérisé en ce que l'on fait réagir un anhydride de formule générale :
(R₁-CO)₂O
dans laquelle R₁ est défini comme précédemment, en présence d'hydrogène sur un produit de formule générale : 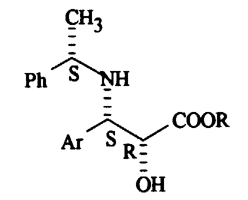 dans laquelle Ar et R sont définis comme précédemment et Ph représente un radical phényle ou *α-* ou β-naphtyle éventuellement substitué par un ou plusieurs atomes ou radicaux, identiques ou différents, choisis parmi les atomes d'halogène et les radicaux alcoxy contenant 1 à 4 atomes de carbone, alcoylthio contenant 1 à 4 atomes de carbone, dialcoylamino dont chaque partie alcoyle contient 1 à 4 atomes de carbone et nitro.

2. Procédé selon la revendication 1 caractérisé en ce que l'on opère en présence d'hydrogène et d'un catalyseur.

3. Procédé selon la revendication 2 caractérisé en ce que le catalyseur est choisi parmi le palladium sur charbon et le dihydroxyde de palladium sur charbon.

4. Procédé selon la revendication 1 caractérisé en ce que l'on opère sous une pression d'hydrogène comprise entre 1 et 50 bars.

5. Procédé selon la revendication 1 caractérisé en ce que l'on opère dans un solvant organique choisi parmi les alcools aliphatiques contenant 1 à 4 atomes de carbone et les hydrocarbures aromatiques et leurs mélanges.

6. Procédé de préparation d'un dérivé de la β-phénylisosérine tels que définis dans la revendication 1 caractérisé en ce que l'on fait réagir un anhydride de formule générale :
(R₁-CO)₂O
dans laquelle R₁ est défini comme dans la revendication 1 avec un produit de formule générale: 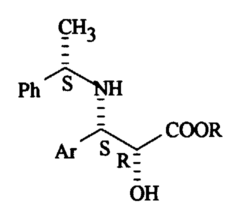 dans laquelle Ar, R et Ph sont définis comme dans la revendication 1 pour obtenir un produit de formule générale : 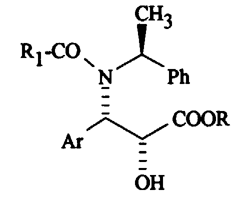 qui est soumis à une hydrogénolyse au moyen d'hydrogène en présence d'un catalyseur.

7. Procédé selon l'une des revendications 1 à 6 pour la préparation de dérivés de la β-phénylisosérine de formule générale : 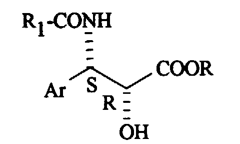 dans laquelle R et R₁ étant définis comme dans la revendication 1, Ar représente un radical phényle ou *α-* ou β-naphtyle éventuellement substitué par un ou plusieurs atomes ou radicaux choisis parmi les atomes d'halogène (fluor, chlore, brome, iode) et les radicaux alcoyles, aryles, aralcoyles, alcoxy, alcoylthio, aryloxy, arylthio, hydroxy, hydroxyalcoyle, mercapto, formyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone et que les radicaux aryles sont des radicaux phényles ou *α-* ou β-naphtyles.

8. Procédé de préparation de dérivés de la classe du taxane de formule générale : 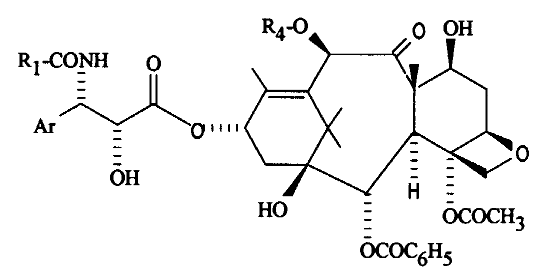 dans laquelle Ar représente un radical phényle ou *α-* ou β-naphtyle éventuellement substitué par un ou plusieurs atomes ou radicaux choisis parmi les atomes d'halogène (fluor, chlore, brome, iode) et les radicaux alcoyles, aryles, aralcoyles, alcoxy, alcoylthio, aryloxy, arylthio, hydroxy, hydroxyalcoyle, mercapto, formyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone et que les radicaux aryles sont des radicaux phényles ou *α-* ou β-naphtyles, R₄ représente un atome d'hydrogène ou un radical acétyle et R₁ représente représente un radical phényle éventuellement substitué par un ou plusieurs atomes ou radicaux, identiques ou différents, choisis parmi les atomes d'halogène et les radicaux alcoyles, hydroxy, alcoxy, alcanoyle, alcanoyloxy, nitro, amino, alcoylamino, dialcoylamino, carbamoyle ou trifluorométhyle, les radicaux alcoyles et les portions alcoyles des autres radicaux contenant 1 à 4 atomes de carbone, ou bien R₁ représente un radical R₂-O- dans lequel R₂ représente :
- un radical alcoyle droit ou ramifié contenant 1 à 8 atomes de carbone, alcényle contenant 3 à 6 atomes de carbone, cycloalcoyle contenant 3 à 6 atomes de carbone, cycloalcényle contenant 4 à 6 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, alcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclyle azoté saturé ou non saturé contenant 4 à 6 chaînons et éventuellement substitué par un ou plusieurs radicaux alcoyles contenant 1 à 4 atomes de carbone
caractérisé en ce que dans une première étape on prépare un dérivé de la β phénylisosérine de fromule (I) dans laquelle R représente l'hydrogène selon la revendication 1 et dans une deuxième étape on protège la fonction hydroxy avec un groupe G3 choisi parmi les groupes méthoxyméthyle, (éthoxy-1) éthyle, benzyloxyméthyle, (β-triméthylsilyléthoxy) méthyle, tétrahydropyrannyle, trichloro-2, 2, 2 éthoxyméthyle, ou trichloro 2, 2, 2 éthoxycarbonyle,
dans une troisième étape on condense le composé obetenu à l'étape 2 avec un noyau taxane de formule (XI) 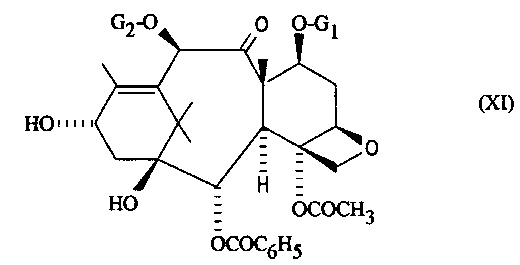 dans laquelle G₁ représente un groupement protecteur de la fonction hydroxy tel qu'un radical trichloro-2,2,2 éthoxycarbonyle ou trialcoylsilyle, dialcoylarylsilyle, alcoyldiarylsilyle ou triarylsilyle dans lequel chaque partie alcoyle contient 1 à 4 atomes de carbone et chaque partie aryle représente de préférence un radical phényle et G₂ représente un radical acétyle ou un groupement protecteur de la fonction hydroxy tel qu'un radical trichloro-2,2,2 éthoxycarbonyle pour donner un produit de formule générale : 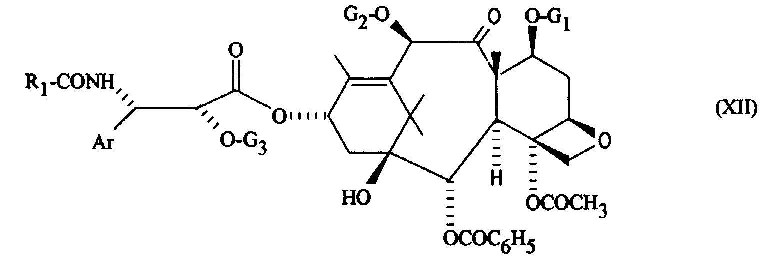 dans laquelle Ar, R₁, G₁, G₂ et G₃ sont définis comme précédemment, suivie du remplacement éventuel des groupements G₁, G₂ et G₃ par des atomes d'hydrogène.

9. Procédé de préparation de dérivés de la classe du taxane de formule générale : 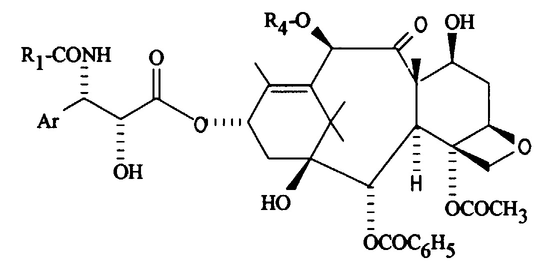 dans laquelle Ar représente un radical phényle ou *α-* ou β-naphtyle éventuellement substitué par un ou plusieurs atomes ou radicaux choisis parmi les atomes d'halogène (fluor, chlore, brome, iode) et les radicaux alcoyles, aryles, aralcoyles, alcoxy, alcoylthio, aryloxy, arylthio, hydroxy, hydroxyalcoyle, mercapto, formyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone et que les radicaux aryles sont des radicaux phényles ou *α-* ou β-naphtyles, R₄ représente un atome d'hydrogène ou un radical acétyle et R₁ représente représente un radical phényle éventuellement substitué par un ou plusieurs atomes ou radicaux, identiques ou différents, choisis parmi les atomes d'halogène et les radicaux alcoyles, hydroxy, alcoxy, alcanoyle, alcanoyloxy, nitro, amino, alcoylamino, dialcoylamino, carbamoyle ou trifluorométhyle, les radicaux alcoyles et les portions alcoyles des autres radicaux contenant 1 à 4 atomes de carbone, ou bien R₁ représente un radical R₂-O- dans lequel R₂ représente :
- un radical alcoyle droit ou ramifié contenant 1 à 8 atomes de carbone, alcényle contenant 3 à 6 atomes de carbone, cycloalcoyle contenant 3 à 6 atomes de carbone, cycloalcényle contenant 4 à 6 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, alcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclyle azoté saturé ou non saturé contenant 4 à 6 chaînons et éventuellement substitué par un ou plusieurs radicaux alcoyles contenant 1 à 4 atomes de carbone
caractérisé en ce que dans une première étape on prépare un dérivé de la β phénylisosérine de formule (I) dans laquelle R représente l'hydrogène selon la revendication 1 dans et dans une deuxième étape on forme un dérivé de l'oxazolidine de formule générale : 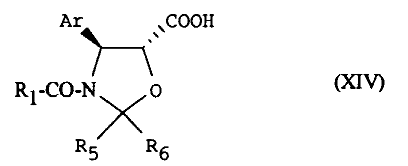 dans laquelle Ar et R₁ sont définis comme précédemment et R₅ et R₆, identiques ou différents, représentent un atome d'hydrogène ou un radical alcoyle contenant 1 à 4 atomes de carbone ou un radical aryle de préférence un radical phényle éventuellement substitué par un ou plusieurs radicaux alcoxy contenant 1 à 4 atomes de carbone, ou bien R₅ représente un radical alcoxy contenant 1 à 4 atomes de carbone ou un radical trihalométhyle tel que trichlorométhyle et R₆ représente un atome d'hydrogène, ou bien R₅ et R₆ forment ensemble avec l'atome de carbone auquel ils sont liés un cycle ayant 4 à 7 chaînons,
dans une deuxième étape on estérifie au moyen de l'acide de formule générale (XIV) un dérivé du taxane de formule générale (XI) 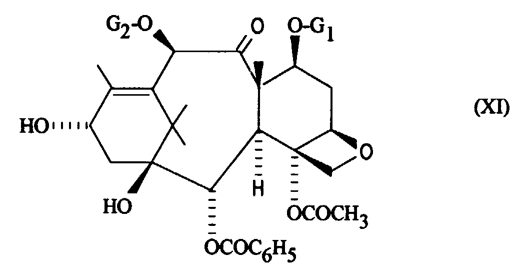 dans laquelle G₁ représente un groupement protecteur de la fonction hydroxy tel qu'un radical trichloro-2,2,2 éthoxycarbonyle ou trialcoylsilyle, dialcoylarylsilyle, alcoyldiarylsilyle ou triarylsilyle dans lequel chaque partie alcoyle contient 1 à 4 atomes de carbone et chaque partie aryle représente de préférence un radical phényle et G₂ représente un radical acétyle ou un groupement protecteur de la fonction hydroxy tel qu'un radical trichloro-2,2,2 éthoxycarbonyle pour donner un produit de formule générale : 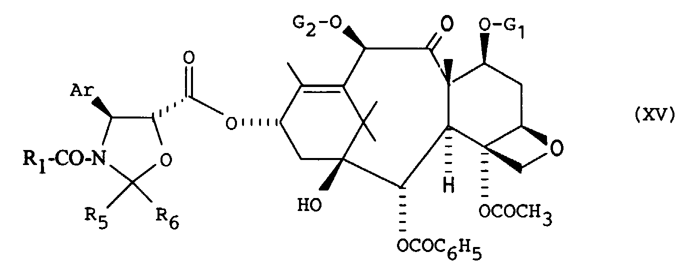 dans laquelle Ar, G₁, G₂, R₁, R₅ et R₆ sont définis comme précédemment, qui est transformé en dérivé du taxane.

## Patentansprüche

1. Verfahren zur Herstellung von β-Phenylisoserinderivaten der allgemeinen Formel: 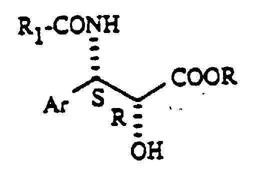 worin:
Ar einen Arylrest bedeutet, und R ein Wasserstoffatom oder einen gegebenenfalls durch einen Phenylrest substituierten Alkylrest bedeutet, und R₁ einen gegebenenfalls substituierten Phenylrest oder einen Rest R₂-O- bedeutet, worin R₂:
- einen geraden oder verzweigten Alkyrest mit 1 bis 8 Kohlenstoffatomen, einen Alkenylrest mit 3 bis 6 Kohlenstoffatomen, einen Cycloalkylrest mit 3 bis 6 Kohlenstoffatomen, einen Cycloalkenylrest mit 4 bis 6 Kohlenstoffatomen, wobei diese Reste gegebenenfalls durch ein oder mehrere Substituenten, ausgewählt aus Halogenatomen und Hydroxygruppen, Alkyloxyresten mit 1 bis 4 Kohlenstoffatomen, Dialkylaminoresten, von denen jeder Alkylteil 1 bis 4 Kohlenstoffatome enthält, Piperidino, Morpholino, 1-Piperazinyl (gegebenenfalls substituiert in Position 4 durch einen Alkylrest mit 1 bis 4 Kohlenstoffatomen oder durch einen Phenylalkylrest, dessen Alkylteil 1 bis 4 Kohlenstoffatome enthält), Cycloalkyl mit 4 bis 6 Kohlenstoffatomen, Alkenyl mit 4 bis 6 Kohlenstoffatomen, Phenyl, Cyano, Carboxy oder Alkyloxycarbonyl, dessen Alkylteil 1 bis 4 Kohlenstoffatome enthält,
- oder einen Phenylrest, der gegebenenfalls durch ein oder mehrere Atome oder Reste, ausgewählt aus Halogenatomen, und Alkylresten mit 1 bis 4 Kohlenstoffatomen oder Alkyloxyresten mit 1 bis 4 Kohlenstoffatomen, substituiert ist,
- oder einen gesättigten oder nichtgesättigten Stickstoffheterozyklus, der 5 oder 6 Kettenglieder enthält und gegebenenfalls durch einen oder mehrere Alkylrest mit 1 bis 4 Kohlenstoffatomen substituiert ist, bedeutet, dadurch **gekennzeichnet**, daß man ein Anhydrid der allgemeinen Formel:
(R₁-CO)₂O
worin R₁ wie vorstehend definiert ist, in Gegenwart von Wasserstoff mit einer Verbindung der allgemeinen Formel: 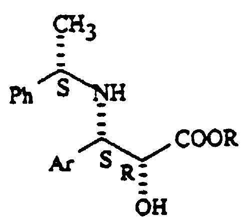 worin Ar und R wie vorstehend definiert sind, und Ph einen Phenylrest oder α- oder β-Naphthylrest, gegebenenfalls substituiert durch ein oder mehrere Atome oder Reste, die gleich oder verschieden sind, ausgewählt aus Halogenatomen und Alkoxyresten mit 1 bis 4 Kohlenstoffatomen, Alkylthioresten mit 1 bis 4 Kohlenstoffatomen, Dialkylaminoresten, deren Alkylteil jeweils 1 bis 4 Kohlenstoffatome enthält, und Nitro bedeutet, umsetzt.

2. Verfahren nach Anspruch 1, dadurch **gekennzeichnet,** daß man in Gegenwart von Wasserstoff und einem Katalysator arbeitet.

3. Verfahren nach Anspruch 2, dadurch **gekennzeichnet,** daß der Katalysator aus Palladium auf Kohle und Palladiumdihydroxid auf Kohle ausgewählt ist.

4. Verfahren nach Anspruch 1, dadurch **gekennzeichnet,** daß man unter einem Wasserstoffdruck zwischen 1 und 50 bar arbeitet.

5. Verfahren nach Anspruch 1, dadurch **gekennzeichnet,** daß man in einem organischen Lösungsmittel, ausgewählt aus aliphatischen Alkoholen mit 1 bis 4 Kohlenstoffatomen und aromatischen Kohlenwasserstoffen und ihren Gemischen, arbeitet.

6. Verfahren zur Herstellung eines β-Phenylisoserinderivats, wie in Anspruch 1 definiert, dadurch **gekennzeichnet,** daß man ein Anhydrid der allgemeinen Formel
(R₁-CO)₂O
worin R₁ wie in Anspruch 1 definiert ist, mit einer Verbindung der allgemeinen Formel 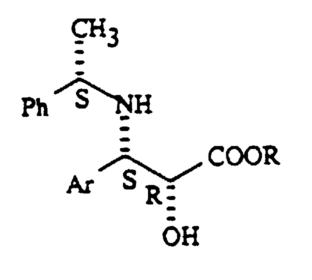 worin Ar, R und Ph wie in Anspruch 1 definiert sind, umsetzt, wodurch eine Verbindung der allgemeinen Formel: 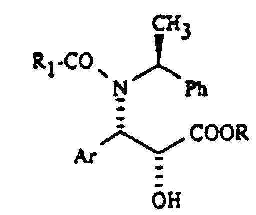 erhalten wird, die einer Hydrogenolyse mit dem Wasserstoff in Gegenwart eines Katalysators unterworfen wird.

7. Verfahren nach einem der Ansprüche 1 bis 6 zur Herstellung von β-Phenylisoserinderivaten der allgemeinen Formel: 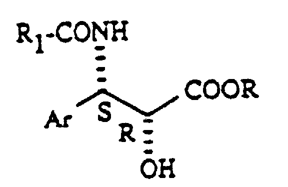 worin R und R₁ wie in Anspruch 1 definiert sind, Ar einen Phenyl- oder α- oder β-Naphthylrest, gegebenenfalls substituiert durch ein oder mehrere Atome oder Reste, ausgewählt aus Halogenatomen (Fluor, Chlor, Brom, Iod), Alkyl-, Aryl-, Aralkyl-, Alkoxy-, Alkylthio-, Aryloxy-, Arylthio-, Hydroxy-, Hydroxyalkyl-, Mercapto-, Formyl-, Acylamino-, Aroylamino-, Alkoxycarbonylamino-, Amino-, Alkylamino-, Dialkylamino-, Carboxy-, Alkoxycarbonyl-, Carbamoyl-, Dialkylcarbamoyl-, Cyano-, Nitro- und Trifluormethylresten, bedeutet, mit der Maßgabe, daß die Alkylreste und die Alkylteile der anderen Reste 1 bis 4 Kohlenstoffatome enthalten und daß die Arylreste Phenyl- oder α - oder β-Naphthylreste sind.

8. Verfahren zur Herstellung von Derivaten der Taxanklasse der allgemeinen Formel: 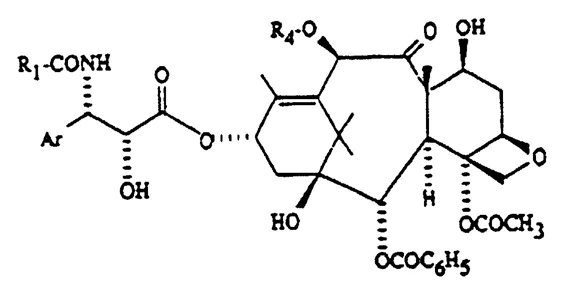 worin Ar einen Phenylrest oder α- oder β-Naphthylrest, gegebenenfalls substituiert durch ein oder mehrere Atome oder Reste, ausgewählt aus Halogenatomen (Fluor, Chlor, Brom, Iod) und Alkyl-, Aryl-, Aralkyl-, Alkoxy-, Alkylthio-, Aryloxy-, Arylthio-, Hydroxy-, Hydroxyalkyl-, Mercapto-, Formyl-, Acylamino-, Aroylamino-, Alkoxycarbonylamino-, Amino-, Alkylamino-, Dialkylamino-, Carboxy-, Alkoxycarbonyl-, Carbamoyl-, Dialkylcarbamoyl-, Cyano-, Nitro- und Trifluormethylresten, bedeutet, mit der Maßgabe, daß die Alkylreste und die Alkylteile der anderen Reste 1 bis 4 Kohlenstoffatome enthalten, und daß die Arylreste Phenyl- oder α- oder β-Naphthylreste sind, R₄ ein Wasserstoffatom oder einen Acetylrest bedeutet, und R₁ einen Phenylrest, gegebenenfalls substituiert durch ein oder mehrere Atome oder Reste, die gleich oder verschieden sind, ausgewählt aus Halogenatomen und Alkyl-, Hydroxy-, Alkoxy-, Alkanoyl-, Alkanoyloxy-, Nitro-, Amino-, Alkylamino-, Dialkylamino-, Carbamoyl- oder Trifluormethylresten, bedeutet, wobei die Alkylreste und die Alkylteile der anderen Reste 1 bis 4 Kohlenstoffatome enthalten oder R₁ einen Rest R₂-O- bedeutet, worin R₂
- einen geraden oder verzweigten Alkylrest mit 1 bis 8 Kohlenstoffatomen, Alkenylrest mit 3 bis 6 Kohlenstoffatomen, Cycloalkylrest mit 3 bis 6 Kohlenstoffatomen, Cycloalkenylrest mit 4 bis 6 Kohlenstoffatomen, wobei diese Reste gegebenenfalls durch ein oder mehrere Substituenten, ausgewählt aus Halogenatomen und Hydroxy-, Alkyloxy- mit 1 bis 4 Kohlenstoffatomen, Dialkylamino-, deren Alkylteile jeweils 1 bis 4 Kohlenstoffatome enthält, Piperidino-, Morpholino-, 1-Piperazinyl- (gegebenenfalls substituiert in Position 4 durch einen Alkylrest mit 1 bis 4 Kohlenstoffatomen oder durch einen Phenylalkylrest, dessen Alkylteil 1 bis 4 Kohlenstoffatome enthält), Cycloalkyl- mit 3 bis 6 Kohlenstoffatomen, Alkenyl- mit 4 bis 6 Kohlenstoffatomen, Phenyl-, Cyano-, Carboxy- oder Alkyloxycarbonylresten, dessen Alkylteil 1 bis 4 Kohlenstoffatome enthält,
- oder einen Phenylrest, gegebenenfalls substituiert durch ein oder mehrere Atome oder Reste, ausgewählt aus Halogenatomen und Alkylresten mit 1 bis 4 Kohlenstoffatomen oder Alkyloxy mit 1 bis 4 Kohlenstoffatomen,
- oder einen Stickstoffheterozyklusrest, der gesättigt oder nichtgesättigt ist und 4 bis 6 Kettenglieder enthält, und gegebenenfalls durch ein oder mehrere Alkylreste mit 1 bis 4 Kohlenstoffatomen substituiert ist, bedeutet,
dadurch **gekennzeichnet,** daß man in einer ersten Stufe ein β-Phenylisoserinderivat der Formel (I) herstellt, worin R ein Wasserstoffatom nach Anspruch 1 bedeutet und in einer zweiten Stufe die Hydroxyfunktion mit einer Gruppe G3, ausgewählt aus den Gruppen Methoxymethyl, (1-Ethoxy)ethyl, Benzyloxymethyl, (β-Trimethylsilylethoxy)-methyl, Tetrahydropyranyl, 2,2,2-Trichlorethoxymethyl oder 2,2,2-Trichlorethoxycarbonyl, schützt,
in einer dritten Stufe die in Stufe 2 erhaltene Verbindung mit einem Taxankern der Formel (XI) 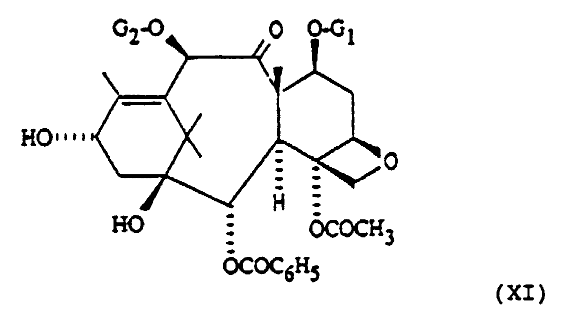 kondensiert, worin G₁ eine Schutzgruppe der Hydroxyfunktion, wie einen 2,2,2-Trichlorethoxycarbonyl- oder Trialkylsilyl-, Dialkylarylsilyl-, Alkyldiarylsilyl- oder Triarylsilylrest, worin jeder Alkylteil 1 bis 4 Kohlenstoffatome enthält, und jeder Arylteil bevorzugt einen Phenylrest bedeutet, bedeutet, und G₂ einen Acetylrest oder eine Schutzgruppe der Hydroxyfunktion, wie einen 2,2,2-Trichlorethoxycarbonylrest bedeutet, wodurch eine Verbindung der allgemeinen Formel: 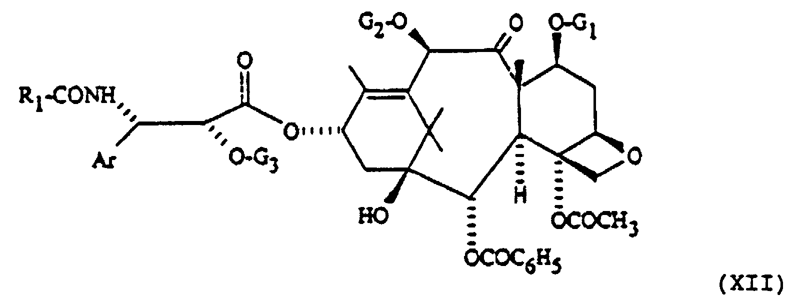 erhalten wird, worin Ar, R₁, G₁, G₂ und G₃ wie vorstehend definiert sind, gefolgt gegebenenfalls von einem Ersatz der Gruppierung G₁, G₂ und G₃ durch Wasserstoffatome.

9. Verfahren zur Herstellung von Derivaten der Taxanklasse der allgemeinen Formel: 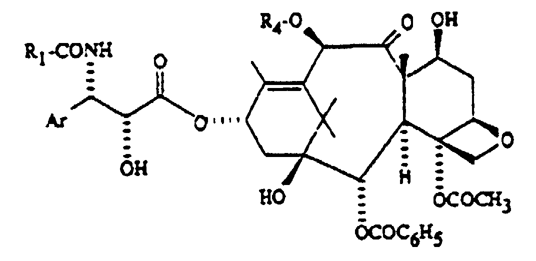 worin Ar einen Phenylrest oder α- oder β-Naphthylrest, gegebenenfalls substituiert durch ein oder mehrere Atome oder Reste, ausgewählt aus Halogenatomen (Fluor, Chlor, Brom, Iod) und Alkyl-, Aryl-, Aralkyl-, Alkoxy-, Alkylthio-, Aryloxy-, Arylthio-, Hydroxy-, Hydroxyalkyl-, Mercapto-, Formyl-, Acylamino-, Aroylamino-, Alkoxycarbonylamino-, Amino-, Alkylamino-, Dialkylamino-, Carboxy-, Alkoxycarbonyl-, Carbamoyl-, Dialkylcarbamoyl-, Cyano-, Nitro- und Trifluormethylresten, mit der Maßgabe, daß die Alkylreste und die Alkylanteile der anderen Reste 1 bis 4 Kohlenstoffatome enthalten, und daß die Arylreste Phenylreste oder α- oder β-Naphthylreste sind, R₄ ein Wasserstoffatom oder einen Acetylrest bedeutet und R₁ einen Phenylrest, gegebenenfalls substituiert durch ein oder mehrere Atome oder Reste, die gleich oder verschieden sind, ausgewählt aus Halogenatomen und Alkyl-, Hydroxy-, Alkoxy-, Alkanoyl-, Alkanoyloxy-, Nitro-, Amino-, Alkylamino-, Dialkylamino-, Carbamoyl- oder Trifluormethylresten, wobei die Alkylreste und die Alkylanteile der anderen Reste 1 bis 4 Kohlenstoffatome enthalten, bedeutet, oder R₁ einen Rest R₂-O- bedeutet, worin R₂:
- einen geraden oder verzweigten Alkylrest mit 1 bis 8 Kohlenstoffatomen, Alkenylrest mit 3 bis 6 Kohlenstoffatomen, Cycloalkylrest mit 3 bis 6 Kohlenstoffatomen, Cycloalkenylrest mit 4 bis 6 Kohlenstoffatomen, wobei die Reste gegebenenfalls durch ein oder mehrere Substituenten, ausgewählt aus Halogenatomen und Hydroxyresten, Alkyloxyresten mit 1 bis 4 Kohlenstoffatomen, Dialkylaminoresten, wobei jeweils der Alkylteil 1 bis 4 Kohlenstoffatome enthält, Piperidinoresten, Morpholinoresten, 1-Piperazinylresten (gegebenenfalls substituiert in Position 4 durch einen Alkylrest mit 1 bis 4 Kohlenstoffatomen oder durch einen Phenylalkylrest, dessen Alkylteil 1 bis 4 Kohlenstoffatome enthält), Cycloalkylresten mit 3 bis 6 Kohlenstoffatomen, Alkenylresten mit 4 bis 6 Kohlenstoffatomen, Phenylresten, Cyanoresten, Carboxyresten oder Alkyloxycarbonylresten, deren Alkylteil 1 bis 4 Kohlenstoffatome enthält,
- oder einen Phenylrest, der gegebenenfalls durch ein oder mehrere Atome oder Reste, ausgewählt aus Halogenatomen, und Alkylresten mit 1 bis 4 Kohlenstoffatomen oder Alkyloxyresten mit 1 bis 4 Kohlenstoffatomen, substituiert ist,
- oder einen Stickstoffheterocyclus, der gesättigt ist oder nicht, mit 4 bis 6 Kettengliedern und gegebenenfalls substituiert durch ein oder mehrere Alkylreste mit 1 bis 4 Kohlenstoffatomen, bedeutet,
dadurch **gekennzeichnet,** daß man in einer ersten Stufe ein β-Phenylisoserinderivat der Formel (I) herstellt, worin R ein Wasserstoffatom gemäß Anspruch 1 bedeutet, und in einer zweiten Stufe ein Oxazolidinderviat der allgemeinen Formel: 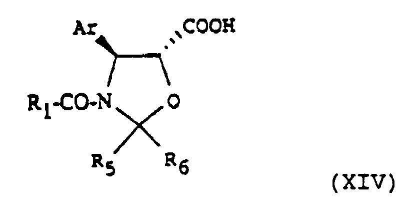 bildet, worin Ar und R₁ wie vorstehend definiert sind, und R₅ und R₆, die gleich oder verschieden sind, ein Wasserstoffatom oder einen Alkylrest mit 1 bis 4 Kohlenstoffatomen oder einen Arylrest, bevorzugt einen Phenylrest, gegebenenfalls substituiert durch ein oder mehrere Alkoxyreste mit 1 bis 4 Kohlenstoffatomen, bedeuten, oder R₅ einen Alkoxyrest mit 1 bis 4 Kohlenstoffatomen oder einen Trihalogenmethylrest, wie Trichlormethyl, bedeutet, und R₆ ein Wasserstoffatom bedeutet, oder R₅ und R₆ zusammen mit dem Kohlenstoffatom, an das sie gebunden sind, einen 4- bis 7gliedrigen Ring bilden,
in einer zweiten Stufe mit einer Säure der allgemeinen Formel (XIV) ein Taxanderivat der allgemeinen Formel (XI) 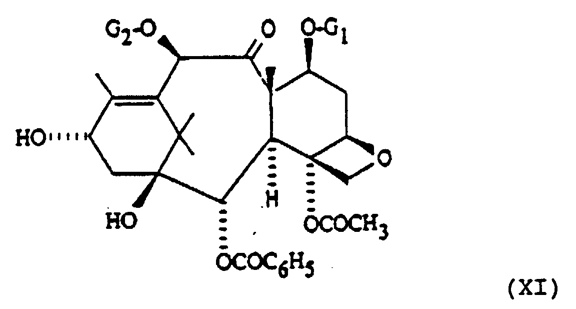 worin G₁ eine Schutzgruppe der Hydroxyfunktion, wie einen 2,2,2-Trichlorethoxycarbonylrest oder Trialkylsilylrest, Dialkylarylsilylrest, Alkyldiarylsilylrest oder Triarylsilylrest bedeutet, worin jeder Alkylteil 1 bis 4 Kohlenstoffatome enthält, und jeder Arylteil bevorzugt eine Phenylgruppe bedeutet, und G₂ einen Acetylrest oder eine Schutzgruppe der Hydroxyfunktion, wie einen 2,2,2-Trichlorethoxycarbonylrest bedeutet, verestert, wodurch eine Verbindung der allgemeinen Formel: 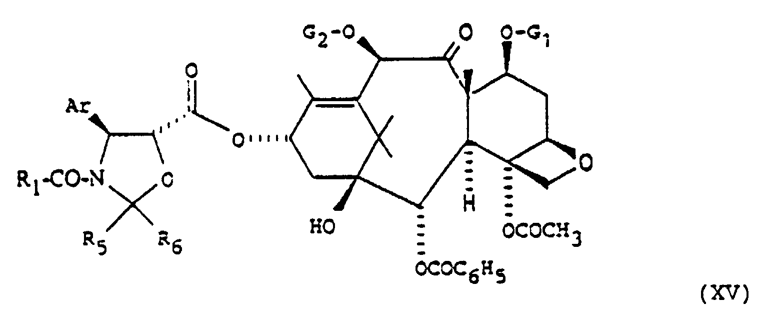 erhalten wird, worin Ar, G₁, G₂, R₁, R₅ und R₆ wie vorstehend definiert sind, die in ein Taxanderivat umgewandelt wird.

## Claims

1. Process for the preparation of β-phenylisoserine derivatives of general formula: 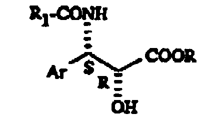 in which Ar represents an aryl radical, R represents a hydrogen atom or an alkyl radical optionally substituted with a phenyl radical, and R₁ represents an optionally substituted phenyl radical or a radical R₂-O- in which R₂ represents
- a straight or branched alkyl radical containing 1 to 8 carbon atoms, an alkenyl radical containing 3 to 6 carbon atoms, a cycloalkyl radical containing 3 to 6 carbon atoms or a cycloalkenyl radical containing 4 to 6 carbon atoms, these radicals being optionally substituted with one or more substituents chosen from halogen atoms and hydroxyl radicals, alkyloxy radicals containing 1 to 4 carbon atoms, dialkylamino radicals in which each alkyl part contains 1 to 4 carbon atoms, piperidino and morpholino radicals, piperazinyl radicals (optionally substituted in the 4-position with an alkyl radical containing 1 to 4 carbon atoms or with a phenylalkyl radical in which the alkyl part contains 1 to 4 carbon atoms), cycloalkyl radicals containing 4 to 6 carbon atoms, alkenyl radicals containing 4 to 6 carbon atoms, phenyl, cyano and carboxyl radicals, and alkyloxycarbonyl radicals in which the alkyl part contains 1 to 4 carbon atoms,
- or a phenyl radical optionally substituted with one or more atoms or radicals chosen from halogen atoms and alkyl radicals containing 1 to 4 carbon atoms, and alkyloxy radicals containing 1 to 4 carbon atoms,
- or a saturated or unsaturated 5- or 6-membered nitrogen-containing heterocyclic radical optionally substituted with one or more alkyl radicals containing 1 to 4 carbon atoms,
characterized in that an anbydride of general formula:
(R₁-CO)₂O
in which R₁ is defined as above, is reacted, in the presence of hydrogen, with a product of general formula: 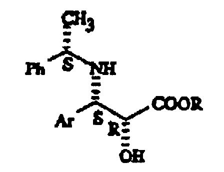 in which Ar and R are defined as above and Ph represents a phenyl or α- or β-naphthyl radical optionally substituted with one or more atoms or radicals, which may be identical or different, chosen from halogen atoms and alkoxy radicals containing 1 to 4 carbon atoms, alkylthio radicals containing 1 to 4 carbon atoms, dialkylamino radicals in which each alkyl part contains 1 to 4 carbon atoms, and nitro radicals.

2. Process according to claim 1, characterized in that it is performed in the presence of hydrogen and a catalyst.

3. Process according to claim 2, characterized in that the catalyst is chosen from palladium-on-charcoal and palladium dihydroxide-on-charcoal.

4. Process according to claim 1, characterized in that it is performed at a hydrogen pressure of between 1 and 50 bar.

5. Process according to claim 1, characterized in that it is performed in an organic solvent chosen from aliphatic alcohols containing 1 to 4 carbon atoms and aromatic hydrocarbons and mixtures thereof.

6. Process for the preparation of a β-phenyl-isoserine derivative as defined in claim 1, characterized in that an anhydride of general formula;
(R₁-CO)₂O
in which R₁ is defined as in claim 1, is reacted with a product of general formula: 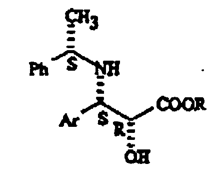 in which Ar, R and Ph are defined as in claim 1, in order to obtain a product of general formula: 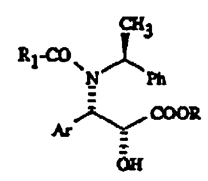 which product is subjected to a hydrogenolysis using hydrogen in the presence of a catalyst.

7. Process according to one of claims 1 to 6, for the preparation of β-phenylisoserine derivatives of general formula; 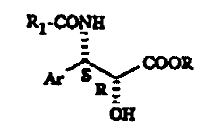 in which, R and R₁ being defined as in claim 1, Ar represents a phenyl or α- or β-naphthyl radical optionally substituted with one or more atoms or radicals chosen from halogen atoms (fluorine, chlorine, bromine and iodine) and alkyl, aryl, aralkyl, alkoxy, alkylthio, aryloxy, arylthio, hydroxyl, hydroxyalkyl, mercapto, formyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms and that the aryl radicals are phenyl or α- or β-naphthyl radicals.

8. Process for the preparation of derivatives of the taxane class, of general formula: 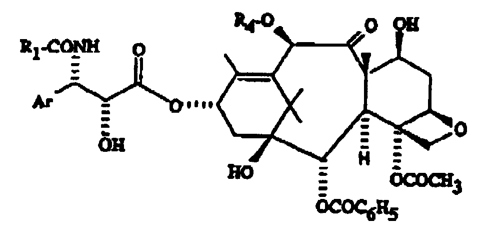 in which Ar represents a phenyl or α- or β-naphthyl radical optionally substituted with one or more atoms or radicals chosen from halogen atoms (fluorine, chlorine, bromine and iodine) and alkyl, aryl, aralkyl, alkoxy, alkylthio, aryloxy, arylthio, hydroxyl, hydroxyalkyl, mercapto, formyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms and that the aryl radicals are phenyl or α- or β-naphthyl radicals, R₄ represents a hydrogen atom or an acetyl radical and R₁ represents a phenyl radical optionally substituted with one or more atoms or radicals, which may be identical or different, chosen from halogen atoms and alkyl, hydroxyl, alkoxy, alkanoyl, alkanoyloxy, nitro, amino, alkylamino, dialkylamino, carbamoyl and trifluoromethyl radicals, the alkyl radicals and the alkyl portions of the other radicals containing 1 to 4 carbon atoms, or alternatively R₁ represents a radical R₂-O- in which R₂ represents:
- a straight or branched alkyl radical containing 1 to 8 carbon atoms, an alkenyl radical containing 3 to 6 carbon atoms, a cycloalkyl radical containing 3 to 6 carbon atoms or a cycloalkenyl radical containing 4 to 6 carbon atoms, these radicals being optionally substituted with one or more substituents chosen from halogen atoms and hydroxyl radicals, alkyloxy radicals containing 1 to 4 carbon atoms, dialkylamino radicals in which each alkyl part contains 1 to 4 carbon atoms, piperidino and morpholino radicals, 1-piperazinyl radicals (optionally substituted in the 4-position with an alkyl radical containing 1 to 4 carbon atoms or with a phenylalkyl radical in which the alkyl part contains 1 to 4 carbon atoms), cycloalkyl radicals containing 3 to 6 carbon atoms, alkenyl radicals containing 4 to 6 carbon atoms, phenyl, cyano and carboxyl radicals, and alkyloxycarbonyl radicals in which the alkyl part contains 1 to 4 carbon atoms,
- or a phenyl radical optionally substituted with one or more atoms or radicals chosen from halogen atoms and alkyl radicals containing 1 to 4 carbon atoms, and alkyloxy radicals containing I to 4 carbon atoms,
- or a saturated or unsaturated 4- to 6-membered nitrogen-containing heterocyclic radical optionally substituted with one or more alkyl radicals containing 1 to 4 carbon atoms,
characterized in that, in a first step, a β-phenylisoserine derivative of formula (I) in which R represents hydrogen, according to claim 1, is prepared and, in a second step, the hydroxyl function is protected with a group G3 chosen from methoxymethyl, (1-ethoxy) ethyl, benzyloxymethyl, (β-trimethylsilylethoxy)methyl, tetrahydropyranyl, 2,2,2-trichloroethoxymethyl and 2,2,2-trichloroethoxycarbonyl groups,
in a third step, the compound obtained in step 2 is condensed with a taxane ring-system of formula (XI) 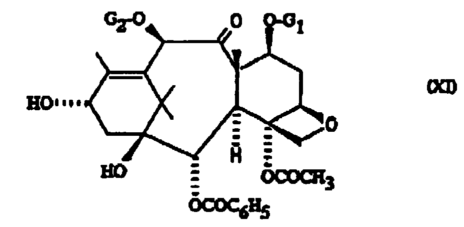 in which G₁ represents a protecting group for the hydroxyl function such as a 2,2,2-trichloroethoxycarbonyl radical or a trialkylsilyl, dialkylarylsilyl, alkyldiarylsilyl or triarylsilyl radical in which each alkyl part contains 1 to 4 carbon atoms and each aryl part preferably represents a phenyl radical, and G₂ represents an acetyl radical or a protecting group .for the hydroxyl function such as a 2,2,2-trichloroethoxycarbonyl radical, in order to give a product of general formula: 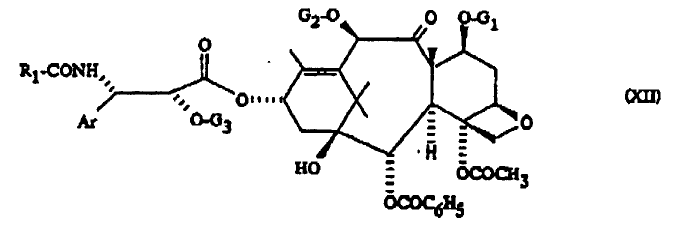 in which Ar, R₁**,** G₁, G₂ and G₃ are defined as above, followed by optional replacement of the groups G₁, G₂ and G₃ by hydrogen atoms.

9. Process for the preparation of derivatives of the taxane class, of general formula: 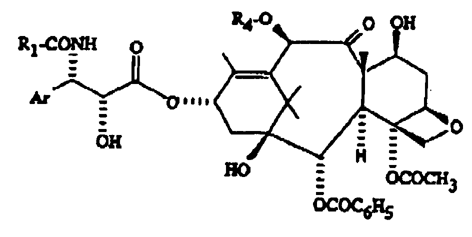 in which Ar represents a phenyl or α- or β-naphthyl radical optionally substituted with one or more atoms or radicals chosen from halogen atoms (fluorine, chlorine, bromine and iodine) and alkyl, aryl, aralkyl, alkoxy, alkylthio, aryloxy, arylthio, hydroxyl, hydroxyalkyl, mercapto, formyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms and that the aryl radicals are phenyl or α- or β-naphthyl radicals, R₄ represents a hydrogen atom or an acetyl radical and R₁ represents a phenyl radical optionally substituted with one or more atoms or radicals, which may be identical or different, chosen from halogen atoms and alkyl, hydroxyl, alkoxy, alkanoyl, alkanoyloxy, nitro, amino, alkylamino, dialkylamino, carbamoyl and trifluoromethyl radicals, the alkyl radicals and the alkyl portions of the other radicals containing 1 to 4 carbon atoms, or alternatively R₁ represents a radical R₂-O- in which R₂ represents:
- a straight or branched alkyl radical containing 1 to 8 carbon atoms, an alkenyl radical containing 3 to 6 carbon atoms, a cycloalkyl radical containing 3 to 6 carbon atoms or a cycloalkenyl radical containing 4 to 6 carbon atoms, these radicals being optionally substituted with one or more substituents chosen from halogen atoms and hydroxyl radicals, alkyloxy radicals containing 1 to 4 carbon atoms, dialkylamino radicals in which each alkyl part contains 1 to 4 carbon atoms, piperidino and morpholine radicals, 1-piperazinyl radicals (optionally substituted in the 4-position with an alkyl radical containing 1 to 4 carbon atoms or with a phenylalkyl radical in which the alkyl part contains 1 to 4 carbon atoms), cycloalkyl radicals containing 3 to 6 carbon atoms, alkenyl radicals containing 4 to 6 carbon atoms, phenyl, cyano and carboxyl radicals, and alkyloxycarbonyl radicals in which the alkyl part contains 1 to 4 carbon atoms.
- or a phenyl radical optionally substituted with one or more atoms or radicals chosen from halogen atoms and alkyl radicals containing 1 to 4 carbon atoms, and alkyloxy radicals containing 1 to 4 carbon atoms,
- or a saturated or unsaturated 4- to 6-membered nitrogen-containing heterocyclic radical optionally substituted with one or more alkyl radicals containing 1 to 4 carbon atoms,
characterized in that, in a first step, a β-phenylisoserine derivative of formula (I) in which R represents hydrogen, according to claim 1, is prepared and, in a second step, an oxazolidine derivative is formed, of general formula: 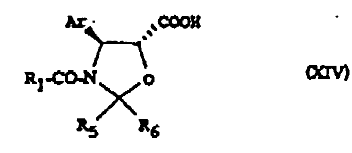 in which Ar = R₁ are defined as above and R₅ and R₆, which may be identical or different, represent a hydrogen atom or an alkyl radical containing 1 to 4 carbon atoms or an aryl radical, preferably a phenyl radical optionally substituted with one or more alkoxy radicals containing 1 to 4 carbon atoms, or alternatively R₅ represents an alkoxy radical containing 1 to 4 carbon atoms or a trihalomethyl radical such as trichloromethyl and R₆ represents a hydrogen atom, or alternatively R₅ and R₆ form, together with the carbon atom to which they are attached, a 4- to 7-membered ring,
in a second step, a taxane derivative of general formula (XI) 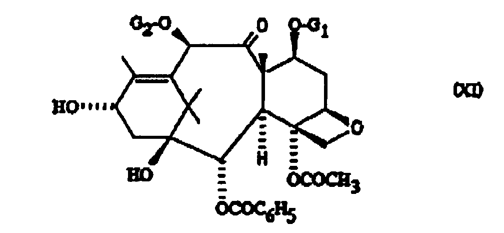 in which G₁ represents a protecting group for the hydroxyl function such as a 2,2,2-trichloroethoxycarbonyl radical or a trialkylsilyl, dialkylarylsilyl, alkyldiarylsilyl or triarylsilyl radical in which each alkyl part contains 1 to 4 carbon atoms and each aryl part preferably represents a phenyl radical, and G₂ represents an acetyl radical or a protecting group for the hydroxyl function such as a 2,2,2-trichloroethoxycarbonyl radical, is esterified using the acid of general formula (XIV) in order to give a product of general formula: 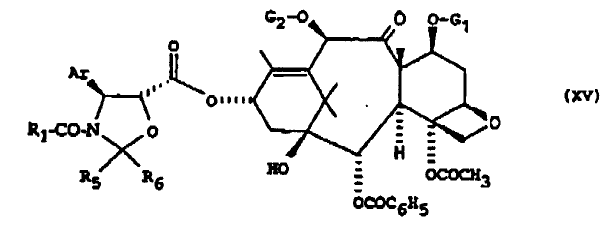 in which Ar, G₁, G₂, R₁, R₅ and R₆ are defined as above, which product is converted into a taxane derivative.
